# EUROPÄISCHE PATENTSCHRIFT

(11) **EP 2 883 863 B1**
(45) Veröffentlichungstag und Bekanntmachung des Hinweises auf die Patenterteilung: **03.05.2017**
(21) Anmeldenummer: 13196584.0
(22) Anmeldetag: 11.12.2013
(51) Int. Cl.: C07C 209/72, C07C 211/36

(54) **Verfahren zur Hydrierung von 4,4'-Methylendianilin**
Process for the preparation of 4,4'-dimethylaniline
Procédé de preparation de 4,4'-dimethylaniline

(43) Veröffentlichungstag der Anmeldung: 17.06.2015
(73) Patentinhaber: BASF SE, 67056 Ludwigshafen am Rhein (DE)
(72) Erfinder: Weickgenannt, Andreas, 68309 Mannheim (DE); Schaack, Bernd Bastian, 64625 Bensheim (DE); Wucher, Barbara, 69514 Laudenbach (DE); Panchenko, Alexander, 67071 Ludwigshafen (DE); Hettche, Frank, 69469 Weinheim (DE); Bock, Martin, 67063 Ludwigshafen (DE); Tan, Aik Meam, 03898 Singapore (SG); Dahmen, Kirsten, 67098 Bad Dürkheim (DE)

(56) Entgegenhaltungen:
- EP-B1- 1 337 331
- WO-A1-2009/153123
- WO-A2-2009/090179

## Beschreibung

Die vorliegende Erfindung betrifft ein Verfahren zur Hydrierung von 4,4'-Methylendianilin (MDA) und/oder Polymer-MDA mit Wasserstoff in Gegenwart eines Katalysators, wobei der Katalysator Ruthenium auf einem Zirkoniumoxid-Trägermaterial enthält, sowie die Verwendung eines Katalysators enthaltend Ruthenium auf einem Zirkoniumoxid-Trägermaterial zur Hydrierung von 4,4'-Methylendianilin (MDA) und/oder Polymer-MDA.

Verfahren zur Hydrierung organischer Verbindungen, insbesondere zur Hydrierung von aromatischen Verbindungen zu den entsprechenden Cyclohexan-Derivaten sind aus dem Stand der Technik bereits bekannt.

WO 2009/153123 A1 offenbart ein kontinuierliches Verfahren und einen Reaktor zur Hydrierung organischer Verbindungen in einem mehrphasigen System in Gegenwart eines homogenen oder heterogenen Katalysators, wobei das Verfahren in zwei Stufen durchgeführt wird. Als mögliche Katalysatoren gemäß diesem Dokument werden heterogene Katalysatoren, beispielsweise enthaltend Edelmetalle wie Platin, Palladium, Ruthenium und Rhodium oder andere Übergangsmetalle, wie Molybdän, Wolfram und Chrom offenbart. Diese heterogenen Katalysatoren können auf Trägermaterialien vorliegen. Entsprechende Trägermaterialien sind beispielsweise Kohlenstoff, Aluminiumoxid, Siliciumdioxid, Zirkoniumdioxid, Zeolithe, Alumosilikate oder Mischungen dieser Trägermaterialien. In Beispiel 1 wurde eine MDA-Schmelze in Gegenwart eines suspendierten Ru-(IV)-oxidhydrat-Katalysators hydriert. Ausführungsbeispiele zur Hydrierung von MDA in Gegenwart von Ru, geträgert auf Zirkoniumoxid, sind in der Anmeldung nicht enthalten. Als Substrate werden in diesem Verfahren bevorzugt aromatische Verbindungen enthaltend Amino-Substituenten eingesetzt, beispielsweise MDA, Polymer-MDA, Anilin, 2,4-Diaminotoluol, 2,6-Diaminotoluol, o-Phenylendiamin, etc. Die heterogenen Katalysatoren werden in Suspension eingesetzt.

DE 19533718 A1 offenbart ein Verfahren zur Hydrierung von aromatischen Verbindungen, in denen mindestens eine Aminogruppe an einen aromatischen Kern gebunden ist. Dazu kann ein heterogener Katalysator enthaltend Ruthenium und gegebenenfalls mindestens ein Metall der I-, VII- oder VIII-Nebengruppe verwendet werden. Als Trägermaterial wird beispielsweise Aluminiumoxid, Siliciumdioxid, Titandioxid oder Zirkoniumdioxid, vorzugsweise Aluminiumdioxid oder Zirkoniumdioxid, eingesetzt. Als Beispiel angegeben wird lediglich ein Katalysator enthaltend Ruthenium auf dem Trägermaterial Aluminiumoxid, nicht jedoch Zirkoniumoxid.

EP 1337331 B1 offenbart ein Verfahren zur katalytischen Hydrierung von aromatischen oder heteroaromatischen Aminen, dadurch gekennzeichnet, dass das Aktivmetall Ruthenium ist und der Katalysator mindestens ein weiteres Metall der I-, VII-, oder VIII-Nebengruppe enthält und diese auf einem Trägermaterial aufgebracht sind, das eine BET(N₂)-Oberfläche von weniger als 10 m²/g aufweist. Als aromatische Verbindungen werden beispielsweise 4,4'-MDA und Isomeren hiervon eingesetzt.

EP 0111238 B1 offenbart ein Verfahren zur katalytischen Hydrierung von 4,4'-MDA, dadurch gekennzeichnet, dass die Hydrierung in Gegenwart von Ruthenium auf einem Trägermaterial und in Anwesenheit von 65 bis 700 Gew%, bezogen auf die Menge Ruthenium, einer mäßigenden Verbindung durchgeführt wird, die aus der Gruppe der Nitrate und Sulfate der Alkalimetalle und der Nitrate der Erdalkalimetalle ausgewählt ist. Ein solcher Zusatz wird in dem erfindungsgemäßen Verfahren nicht wissentlich zugesetzt.

In EP 1366812 B1 wird ein Verfahren zur Hydrierung eines aromatischen Amins offenbart, dass in Gegenwart des Aktivmetalls Ruthenium auf einem Trägermaterial durchgeführt wird. Das Verfahren ist dadurch gekennzeichnet, dass die BET-Oberfläche des verwendeten Trägermaterials im Bereich von größer 30 m²/g bis kleiner 70 m²/g liegt. Als Trägermaterialien werden unter anderem Aluminiumoxid, Siliciumoxid, Titanoxid und Zirkoniumoxid offenbart. In den Beispielen wird lediglich Aluminumoxid als Trägermaterial eingesetzt, nicht jedoch Zirkoniumoxid.

WO 2011/003899 A1 offenbart ein Verfahren zur Hydrierung organischer Verbindungen, beispielsweise aromatischer Verbindungen. Dazu kann ein heterogener Katalysator enthaltend Edelmetalle, wie Platin, Palladium, Ruthenium, Osmium, Iridium und Rhodium oder andere Übergangsmetalle eingesetzt werden. Als Trägermaterialien werden beispielsweise Aluminiumoxid, Siliciumdioxid, Titandioxid und Aktivkohle genannt, nicht jedoch Zirkoniumoxid.

WO2009/090179 offenbart ein Verfahren zur Hydrierung von aromatischen Aminen mit Wasserstoff in Gegenwart eines Ru-Katalysators, welcher, u.a. Zirkoniumoxid-Trägermaterial enthält.

Bei der Hydrierung, d. h. bei der Kernhydrierung, von 4,4'-Methylendianilin zu 4,4'-Diaminodicyclohexylmethan können drei Isomere, nämlich trans,trans-4,4'-Diaminodicyclohexylmethan, cis,trans-4,4'-Diaminodicyclohexylmethan und cis,cis-4,4'-Diaminodicyclohexylmethan entstehen. Die genannten Isomere bzw. ihr Anteil in der erhaltenen Mischung haben einen deutlichen Einfluss auf die physikalischen Eigenschaften, beispielsweise den Schmelzpunkt, der Mischung. Um einen möglichst niedrigen Schmelzpunkt zu erhalten, ist es beispielsweise vorteilhaft, wenn der Anteil des trans,trans-Produktes in der erhaltenen Produktmischung möglichst niedrig ist.

Polymer-MDA ist dem Fachmann an sich bekannt. Insbesondere versteht der Fachmann darunter oligomere oder polymere Additionsprodukte von 4,4'-Methylendianilin, beispielsweise enthaltend 2 bis 100, insbesondere 3 bis 7 Wiederholungseinheiten 4,4'-Methylendianilin. Bei der erfindungsgemäßen Hydrierung werden aus dem eingesetzten Polymer-MDA die entsprechenden kernhydrierten Oligomere oder Polymere. Dabei können für jede einzelne 4,4'-Methylendianilin-Einheit die entsprechenden trans,trans-, cis-trans- bzw. cis,cis-Isomere erhalten werden. Bevorzugt wird durch das erfindungsgemäße Verfahren eine entsprechende oligomere oder polymere kerhydrierte verbindung erhalten, die einen möglichst geringen Anteil an trans,trans-Wiederholungseinheiten aufweisen. Aufgabe der vorliegenden Erfindung ist es daher, ein Verfahren zur Hydrierung von 4,4'-Methylendianilin zu 4,4'-Diaminodicyclohexylmethan und/oder von Polymer-MDA zu der entsprechenden kernhydrierten Verbindung bereitzustellen, in dem ein Katalysator eingesetzt wird, der zum einen über einen langen Zeitraum eine besonders hohe Aktivität aufweist, so dass über einen langen Zeitraum ein hoher Umsatz erzielt werden kann, und des Weiteren ein Produktgemisch, d. h. eine Mischung der drei Isomeren trans,trans-4,4'-Diaminodicyclohexylmethan, cis,trans-4,4'-Diaminodicyclohexylmethan und cis,cis-4,4'-Diaminodicyclohexylmethan, bzw. entsprechender oligomerer bzw. polymerer kernhydrierten Verbindungen zugänglich macht, welches sich durch einen besonders niedrigen Anteil des trans,trans-Isomers, und damit durch einen niedrigen Schmelzpunkt auszeichnet. Des Weiteren soll erfindungsgemäß im Festbett oder in Suspension gearbeitet werden können, ohne dass die aus Verfahren des Standes der Technik bekannten Nachteile auftreten, beispielsweise Koksbildung bzw. Sinterung des eingesetzten Katalysators bei einer Festbettreaktion.

Diese Aufgaben werden gelöst durch das erfindungsgemäße Verfahren zur Hydrierung von 4,4'-Methylendianilin und/oder Polymer-MDA mit Wasserstoff in Gegenwart eines Katalysators, wobei der Katalysator Ruthenium auf einem Zirkoniumoxid-Trägermaterial enthält. Die Aufgaben werden des Weiteren gelöst durch die Verwendung eines Katalysators enthaltend Ruthenium auf einem Zirkoniumoxid-Trägermaterial zur Hydrierung von 4,4'-Methylendianilin und/oder Polymer-MDA.

Die vorliegende Erfindung zeichnet sich dadurch aus, dass ein Katalysator eingesetzt wird, der Ruthenium auf einem Zirkoniumoxid-Trägermaterial enthält, um 4,4'-Methylendianilin zu 4,4'-Diaminodicyclohexylmethan und/oder Polymer-MDA zu der entsprechenden kernhydrierten Verbindung zu hydrieren, wobei das trans,trans-Isomer der gewünschten Verbindung in einem geringen Anteil vorliegt.

In dem erfindungsgemäßen Verfahren wird die dem Fachmann bekannte Verbindung 4,4'-Methylendianilin (MDA) (I) 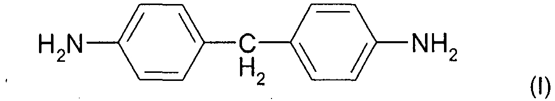 eingesetzt. Diese Verbindung ist kommerziell erhältlich oder kann nach dem Fachmann bekannten Verfahren, beispielsweise beschrieben in WO 2008/083997, hergestellt werden.

Erfindungsgemäß kann 4,4'-Methylendianilin in reiner Form eingesetzt werden. Es ist erfindungsgemäß auch möglich, dass das eingesetzte 4,4'-Methylendianilin neben dem gewünschten Isomer auch 2,4'-Methylendianilin (II) 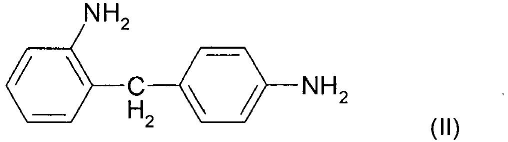 und gegebenenfalls weitere Isomere zu einem Anteil von bis 30 Gew.-%, bevorzugt bis zu 7 Gew.-%, jeweils bezogen auf die Gesamtmenge an eingesetztem Methylendianilin enthält.

Durch das erfindungsgemäße Verfahren wird 4,4'-Methylendianilin kernhydriert, d. h. es werden die entsprechenden isomeren Dicyclohexyl-Derivate erhalten. Die einzelnen Isomere von 4,4'-Diaminodicyclohexylmethan, d. h. trans,trans-4,4'-Diaminodicyclohexylmethan (IIIa), cis,trans-4,4'-Diaminodicyclohexylmethan (IIIb) und cis,cis-4,4'-Diaminodicyclohexylmethan (IIIc), sind im Folgenden abgebildet.

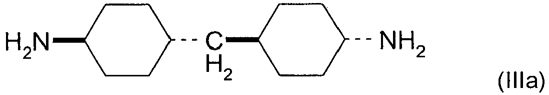

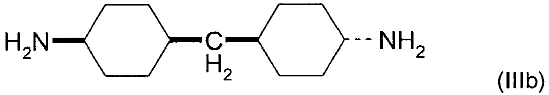

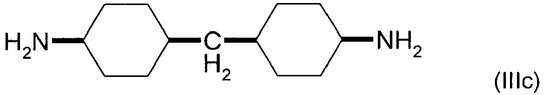

Durch das erfindungsgemäße Verfahren wird bevorzugt ein Produkt erhalten, das ohne weitere Aufreinigung des Reaktoraustrages das trans,trans-Isomer in einer Menge von weniger als 25 Gew.-%, bevorzugt weniger als 23 Gew.-% enthält, jeweils bezogen auf die gesamte Menge des erhaltenen Produktes, wobei der verbleibende Anteil auf die cis,trans- und/oder cis,cis-Isomere, und gegebenenfalls auf Hydrierprodukte von 2,4'-Methylendianilin entfällt.

In einer bevorzugten Ausführungsform des erfindungsgemäßen Verfahrens wird als Produkt eine Mischung erhalten, enthaltend die Isomeren von 4,4'-Diaminodicyclohexylmethan, wobei das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden Isomeren, vorliegen, wobei die Summe der vorliegenden Isomeren immer 100 Gew.-% ergibt.

In einer weiteren bevorzugten Ausführungsform des erfindungsgemäßen Verfahrens wird ausgehend von Polymer-MDA als Produkt eine Mischung von entsprechenden oligomeren oder polymeren, kernhydrierten Verbindungen erhalten, enthaltend als Wiederholungseinheiten die Isomeren von 4,4'-Diaminodicyclohexylmethan, wobei als Wiederholungseinheiten das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden isomeren Wiederholungseinheiten, vorliegen, wobei die Summe der vorliegenden Isomeren immer 100 Gew.-% ergibt.

Die Anteile der einzelnen in dem erfindungsgemäß erhaltenen Produkt enthaltenen Isomeren können durch dem Fachmann bekannte analytische Methoden bestimmt werden. Eine bevorzugte analytische Methode ist die dem Fachmann bekannte Gaschromatographie (GC).

Ein erfindungsgemäß bevorzugt erhaltenes Produkt mit den oben angegebenen niedrigen Anteilen an trans,trans-Isomer weist einen Schmelzpunkt von weniger als 40 °C, bevorzugt weniger als 30 °C, besonders bevorzugt weniger als 22 °C, auf. Eine bevorzugte Untergrenze für den Schmelzpunkt ist beispielsweise 0 °C.

Das erfindungsgemäße Verfahren kann im Allgemeinen kontinuierlich oder diskontinuierlich durchgeführt werden. In einer bevorzugten Ausführungsform betrifft die vorliegende Erfindung das erfindungsgemäße Verfahren, wobei es kontinuierlich durchgeführt wird.

Das erfindungsgemäße Verfahren kann im Allgemeinen in Suspension oder im Festbett durchgeführt werden.

Die vorliegende Erfindung betrifft daher bevorzugt das erfindungsgemäße Verfahren, wobei es in Suspension oder im Festbett durchgeführt wird.

Bei der diskontinuierlichen Reaktionsführung kann die Hydrierung beispielsweise in einem Rührkessel bzw. Rührautoklaven, einem Schlaufenreaktor, einem Strahlschlaufenreaktor, einer Blasensäule oder einem Festbettreaktor mit Umpumpkreislauf durchgeführt werden. Bevorzugt wird die diskontinuierliche Hydrierung in einem Rührkessel bzw. Rührautoklaven durchgeführt.

Bei der kontinuierlichen Reaktionsführung wird die Hydrierung üblicherweise in einem kontinuierlich betriebenen Rührkesselreaktor, einem kontinuierlich betriebenen Schlaufenreaktor, einem kontinuierlich betriebenen Strahlschlaufenreaktor, einer kontinuierlich betriebenen Blasensäule oder einem kontinuierlich betriebenen Festbettreaktor mit Umpumpkreislauf oder einer Rührkesselkaskade durchgeführt.

Vorzugsweise führt man das Verfahren in Rieselreaktoren oder in gefluteter Fahrweise nach der Festbettfahrweise durch, beispielsweise gemäß der WO 2008/015135 A1. Der Wasserstoff kann dabei sowohl im Gleichstrom mit der Lösung des zu hydrierenden Edukts als auch im Gegenstrom über den Katalysator geleitet werden.

Geeignete Apparaturen zur Durchführung einer Hydrierung am Katalysatorfließbett und am Katalysatorfestbett sind aus dem Stand der Technik bekannt, z.B. aus Ullmanns Enzyklopädie der Technischen Chemie, 4. Auflage, Band 13, S. 135 ff., sowie aus P. N. Rylander, "Hydrogenation and Dehydrogenation" in Ullmann's Encyclopedia of Industrial Chemistry, 5th ed. on CD-ROM.

Um einen vollständigen Umsatz zu erzielen, kann eine Nachreaktion des Hydrieraustrags erfolgen. Dazu kann der Hydrieraustrag im Anschluss an das Hydrierverfahren in der Gasphase oder in der Flüssigphase im geraden oder umgepumpten Durchgang, durch einen oder mehrere nachgeschaltete Reaktoren geleitet werden. Der Reaktor kann bei Flüssigphasenhydrierung in Rieselfahrweise betrieben oder geflutet betrieben werden. Der Reaktor ist mit dem erfindungsgemäßen oder mit einem anderen, dem Fachmann bekannten, Katalysator befüllt.

Geeignete Reaktoren für die Durchführung des erfindungsgemäßen Verfahrens in Suspensionsfahrweise sind dem Fachmann an sich bekannt, beispielsweise Rührkessel oder Blasensäule. Erfindungsgemäß ist es auch möglich, dass in einer Kaskade mehrerer hintereinander geschalteter Suspensionsreaktoren gearbeitet wird, beispielsweise in einer Rührkesselkaskade oder einer Blasensäulenkaskade, beispielsweise mit jeweils mindestens drei entsprechenden, hintereinandergeschalteten Reaktoren.

Das erfindungsgemäße Verfahren wird im Allgemeinen bei einem Druck von 50 bis 500 bar durchgeführt, vorzugsweise bei einem Druck von 60 bis 300 bar.

Die vorliegende Erfindung betrifft daher bevorzugt das erfindungsgemäße Verfahren, wobei es bei einem Druck von 60 bis 300 bar durchgeführt wird.

Da das erfindungsgemäße Verfahren besonders bevorzugt ohne Zugabe eines weiteren Gases neben Wasserstoff durchgeführt wird, wird der Verfahrensdruck bevorzugt durch den Wasserstoff-Partialdruck bestimmt. Die vorliegende Erfindung betrifft daher insbesondere bevorzugt das erfindungsgemäße Verfahren, wobei es bei einem Wasserstoffdruck von 50 bis 500 bar, bevorzugt 60 bis 300 bar, durchgeführt wird.

Erfindungsgemäß wird das Verfahren im Allgemeinen bei einer Temperatur von 30 bis 280 °C durchgeführt, vorzugsweise bei einer Temperatur von 60 bis 250 °C.

In der bevorzugten Ausführungsform, dass das Verfahren gemäß der vorliegenden Erfindung im Festbett durchgeführt wird, wird es bevorzugt bei einer Temperatur von 50 bis 190 °C, bevorzugt 70 bis 120 °C, durchgeführt.

Die vorliegende Erfindung betrifft daher bevorzugt das erfindungsgemäße Verfahren, wobei es im Festbett bei einer Temperatur von 50 bis 190 °C, bevorzugt 70 bis 120 °C, durchgeführt wird.

In der weiteren bevorzugten Ausführungsform, dass das Verfahren gemäß der vorliegenden Erfindung in Suspension durchgeführt wird, wird es bevorzugt bei einer Temperatur von 50 bis 190 °C, bevorzugt 100 bis 140 °C, durchgeführt.

Die vorliegende Erfindung betrifft daher bevorzugt das erfindungsgemäße Verfahren, wobei es in Suspension bei einer Temperatur von 50 bis 190 °C, bevorzugt 100 bis 140 °C, durchgeführt wird.

In dem erfindungsgemäßen Verfahren wird Wasserstoff als Hydriermittel eingesetzt.

Der als Hydriermittel eingesetzte Wasserstoff wird in einer bevorzugten Ausführungsform im Überschuss bezogen auf die zu hydrierende Verbindung eingesetzt. Beispielsweise wird Wasserstoff als Hydriermittel in einem 1,01 bis 10-fachen, bevorzugt 1,05 bis 10-fachen, weiter bevorzugt 1- bis 10-fachen, besonders bevorzugt 1,01- bis 5-fachen, beispielsweise 1,1- bis 5-fachen, stöchiometrischen Überschuss eingesetzt. In einer Ausführungsform kann der eingesetzte Wasserstoff als Kreisgas in die Reaktion zurückgeführt werden.

In einer bevorzugten Ausführungsform des erfindungsgemäßen Verfahrens wird technisch reiner Wasserstoff eingesetzt. Im Rahmen der vorliegenden Erfindung wird unter "technisch rein" ein Gehalt an Wasserstoff von mindestens 99,0 Gew.-%, bevorzugt mindestens 99,5 Gew.-%, verstanden.

In einer weiteren erfindungsgemäßen Ausführungsform kann der Wasserstoff auch in Form eines Wasserstoff enthaltenden Gases eingesetzt werden. Möglich sind hierbei beispielsweise Mischungen enthaltend Gase und Inertgase wie Stickstoff, Helium, Neon, Argon, Ammoniak und/oder Kohlendioxid. Als Wasserstoff enthaltende Gase können beispielsweise Reformerabgase, Raffineriegase usw. verwendet werden. Diese Wasserstoff enthaltenden Gase weisen einen Wasserstoffgehalt von beispielsweise 10 bis 100 Gew.-%, bevorzugt 50 bis 100 Gew.-%, auf.

Das erfindungsgemäße Verfahren kann im Allgemeinen in Gegenwart oder in Abwesenheit wenigstens eines Lösungsmittels durchgeführt werden. Besonders bevorzugt wird das Verfahren in einem organischen Lösungsmittel durchgeführt. In einer weiteren bevorzugten Ausführungsform wird das erfindungsgemäße Verfahren in Substanz, d. h. als Schmelze in Abwesenheit eines Lösungsmittels, durchgeführt.

Der Einsatz von Lösungsmitteln ist beispielsweise vorteilhaft, wenn die organische Verbindung als Feststoff vorliegt und sich nicht oder nur unter großem Aufwand als Schmelze handhaben und fördern lässt. Geeignete Lösungsmittel sind beispielsweise ausgewählt aus der Gruppe bestehend aus Alkoholen, wie Isopropanol, Isobutanol oder t-Butanol, Ethern, wie Diethylether, Glykoldimethylether (Diglyme), Glykoldipropylether (Proglyme), Dioxan oder Tetrahydrofuran, und Mischungen davon. In einer bevorzugten Ausführungsform wird Dioxan oder Proglyme als Lösungsmittel eingesetzt. In einer weiteren erfindungsgemäßen Ausführungsform wird Methyldiaminocyclohexan eingesetzt. In einer weiteren erfindungsgemäßen Ausführungsform wird das bei der Reaktion entstehende Produkt, d. h. 4,4'-Diaminodicyclohexylmethan, insbesondere eine erfindungsgemäße Isomerenmischung enthaltend trans,trans-4,4'-Diaminodicyclohexylmethan, cis,trans-4,4'-Diaminodicyclohexylmethan und cis,cis-4,4'-Diaminodicyclohexylmethan, oder die gebildeten Leichtsieder, zum Beispiel 4-Aminocyclohexylmethylcyclohexan als Lösungsmittel eingesetzt.

Wird das erfindungsgemäße Verfahren in Gegenwart eines Lösungsmittels durchgeführt, so wird dieses im Allgemeinen in einer solchen Menge eingesetzt, so dass eine 2 bis 50 gew.-%ige, bevorzugt 5 bis 40 gew.-%ige, besonders bevorzugt 8 bis 30 gew.-%ige Lösung der zur Hydrierung vorgesehenen organischen Verbindungen vorliegt.

Erfindungsgemäß wird als Katalysator Ruthenium auf einem Zirkoniumoxid-Trägermaterial eingesetzt.

Entsprechende Katalysatoren können durch bekannte Verfahren wie Imprägnierung bzw. Tränkung, beispielsweise beschrieben in A. B. Stiles, Catalyst Manufacture-Laboratory and Commerical Preparations, Marcel Dekker, New York, 1983, oder Aus- bzw. Auffällung, beispielsweise beschrieben in EP 1 106 600, Seite 4, und A. B. Stiles, Catalyst Manufacture, Marcel Dekker Inc., 1983, Seite 15, hergestellt werden.

Zur Herstellung der erfindungsgemäß einzusetzenden Katalysatoren kann so vorgegangen werden, dass auf das Trägermaterial Zirkoniumoxid in Form von Strangpresslingen, Pillen oder Kugeln, beispielsweise mit Durchmessern von etwa 1,5 bis 10 mm, geeignete Verbindungen des Rutheniums, beispielsweise Rutheniumsalze, aufgebracht werden. Anschließend wird im Allgemeinen bei einer Temperatur von 80 bis 180 °C, beispielsweise 120 °C, getrocknet und bei einer Temperatur von 180 bis 450 °C, beispielsweise 180 °C, kalziniert, beide Schritte können auch simultan erfolgen. Für das Aufbringen geeignete Rutheniumsalze sind beispielsweise ausgewählt aus der Gruppe bestehend aus Ruthenium-acetat, -acetylacetonat, -chlorid, - nitrosylnitrat und Mischungen davon.

Ein entsprechend hergestellter Katalysator steht nach dem Trocknen grundsätzlich für den erfindungsgemäßen Einsatz zur Verfügung. In bevorzugter Weise wird er jedoch vor seinem Einsatz, besonders bevorzugt nach Anordnung in dem für die erfindungsgemäße Hydrierung vorgesehenen Reaktor, durch Behandlung mit Wasserstoff bei einer Temperatur von beispielsweise 150 bis 400 °C, aktiviert.

Auf dem erfindungsgemäß eingesetzten Katalysator liegt Ruthenium bevorzugt in einer Gesamtmenge von 0,05 bis 15 Gew.-%, bevorzugt 0,05 bis 12 Gew.-%, besonders bevorzugt 0,1 bis 11 Gew.-%, jeweils bezogen auf das Gesamtgewicht des Katalysators, vor.

Das Trägermaterial Zirkoniumoxid (ZrO₂) liegt erfindungsgemäß bevorzugt in monokliner, tetragonaler, kubischer, amorpher Phase oder einer Mischphase, besonders bevorzugt in monokliner, tetragonaler oder einer Mischphase dieser Modifikationen vor.

Die vorliegende Erfindung betrifft daher bevorzugt das erfindungsgemäße Verfahren, wobei das Zirkoniumoxid-Trägermaterial in monokliner, tetragonaler, kubischer, amorpher Phase oder einer Mischphase dieser Modifikationen vorliegt.

Weiter bevorzugt betrifft die vorliegende Erfindung das erfindungsgemäße Verfahren, wobei das Zirkoniumoxid-Trägermaterial in monokliner, tetragonaler, kubischer, amorpher Phase oder einer Mischphase dieser Modifikationen vorliegt.

Erfindungsgemäß bevorzugt weist das Zirkoniumoxid-Trägermaterial, bevorzugt vor Aufbringen von Ruthenium, eine BET-Oberfläche von 30 bis 300 m²/g, bevorzugt 35 bis 250 m²/g, besonders bevorzugt 50 bis 90 m²/g, auf, jeweils bestimmt durch Stickstoffsorption nach DIN 66131.

Erfindungsgemäß bevorzugt weist das Zirkoniumoxid-Trägermaterial, bevorzugt vor Aufbringen des Ruthenium, ein Porenvolumen von 0,1 bis 1 cm³/g, bevorzugt 0,1 bis 0,9 cm³/g, besonders bevorzugt 0,1 bis 0,9 cm³/g, auf, jeweils bestimmt durch Quecksilberporosimetrie nach DIN 66133.

Erfindungsgemäß bevorzugt weist das Zirkoniumoxid-Trägermaterial des erfindungsgemäßen Katalysators, der in Suspension eingesetzt wird, bevorzugt vor Aufbringen des Ruthenium, ein Porenvolumen von 0,1 bis 1 cm³/g, bevorzugt 0,5 bis 1,0 cm³/g, besonders bevorzugt 0,7 bis 0,9 cm³/g, auf, jeweils bestimmt durch Quecksilberporosimetrie nach DIN 66133.

Erfindungsgemäß bevorzugt weist das Zirkoniumoxid-Trägermaterial des erfindungsgemäßen Katalysators, der im Festbett eingesetzt wird, bevorzugt vor Aufbringen des Ruthenium, ein Porenvolumen von 0,1 bis 1 cm³/g, bevorzugt 0,1 bis 0,6 cm³/g, besonders bevorzugt 0,1 bis 0,5 cm³/g, auf, jeweils bestimmt durch Quecksilberporosimetrie nach DIN 66133.

Erfindungsgemäß bevorzugt weist das Zirkoniumoxid-Trägermaterial eine Stampfdichte, bevorzugt vor Aufbringen des Rutheniums, von 500 bis 2000 kg/m³, bevorzugt 600 bis 1800 kg/m³, besonders bevorzugt 700 bis 1750 kg/m³, auf, jeweils bestimmt in einem Stampfvolumeter STAV2003 der Firma JEL, wobei 2000 mal gestampft wurde.

Erfindungsgemäß besonders bevorzugt weist das Zirkoniumoxid-Trägermaterial, bevorzugt vor Aufbringen des Rutheniums, eine BET-Oberfläche von 30 bis 300 m²/g, bevorzugt 35 bis 250 m²/g, besonders bevorzugt 50 bis 90 m²/g, auf, jeweils bestimmt durch Stickstoffsorption, ein Porenvolumen von 0,1 bis 1 cm³/g, bevorzugt 0,1 bis 0,8 cm³/g, besonders bevorzugt 0,1 bis 0,7 cm³/g, auf, jeweils bestimmt durch Quecksilberporosimetrie und eine Stampfdichte von 500 bis 2000 kg/m³, bevorzugt 600 bis 1750 kg/m³, besonders bevorzugt 700 bis 1500 kg/m³, jeweils bestimmt in einem Stampfvolumeter STAV2003 der Firma JEL, wobei 2000 mal gestampft wurde, auf.

Die vorliegende Erfindung betrifft daher bevorzugt das erfindungsgemäße Verfahren, wobei das Zirkoniumoxid-Trägermaterial, bevorzugt vor Aufbringen des Rutheniums, eine BET-Oberfläche von 30 bis 300 m²/g, bevorzugt 35 bis 250 m²/g, besonders bevorzugt 50 bis 90 m²/g, auf, jeweils bestimmt durch Stickstoffsorption, ein Porenvolumen von 0,1 bis 1 cm³/g, bevorzugt 0,1 bis 0,8 cm³/g, besonders bevorzugt 0,1 bis 0,7 cm³/g, auf, jeweils bestimmt durch Quecksilberporosimetrie und eine Stampfdichte von 500 bis 2000 kg/m³, bevorzugt 600 bis 1800 kg/m³, besonders bevorzugt 700 bis 1500 kg/m³, jeweils bestimmt in einem Stampfvolumeter STAV2003 der Firma JEL, wobei 2000 mal gestampft wurde, aufweist.

Erfindungsgemäß besonders bevorzugt weist das Zirkoniumoxid-Trägermaterial, bevorzugt vor Aufbringen des Rutheniums, eine monokline oder tetragonale Modifikation (bzw. ein Gemisch beider), eine BET-Oberfläche von 30 bis 300 m²/g, bevorzugt 35 bis 250 m²/g, besonders bevorzugt 50 bis 90 m²/g, auf, jeweils bestimmt durch Stickstoffsorption, ein Porenvolumen von 0,1 bis 1 cm³/g, bevorzugt 0,1 bis 0,8 cm³/g, besonders bevorzugt 0,1 bis 0,7 cm³/g, auf, jeweils bestimmt durch Quecksilberporosimetrie und eine Stampfdichte von 500 bis 2000 kg/m³, bevorzugt 600 bis 1800 kg/m³, besonders bevorzugt 700 bis 1500 kg/m³, jeweils bestimmt in einem Stampfvolumeter STAV2003 der Firma JEL, wobei 2000 mal gestampft wurde, auf.

Die vorliegende Erfindung betrifft daher bevorzugt das erfindungsgemäße Verfahren, wobei das Zirkoniumoxid-Trägermaterial, bevorzugt vor Aufbringen des Rutheniums, eine monokline oder tetragonale Modifikation (bzw. ein Gemisch beider), eine BET-Oberfläche von 30 bis 300 m²/g, bevorzugt 35 bis 250 m²/g, besonders bevorzugt 50 bis 90 m²/g, auf, jeweils bestimmt durch Stickstoffsorption, ein Porenvolumen von 0,1 bis 1 cm³/g, bevorzugt 0,1 bis 0,8 cm³/g, besonders bevorzugt 0,1 bis 0,7 cm³/g, auf, jeweils bestimmt durch Quecksilberporosimetrie und eine Stampfdichte von 500 bis 2000 kg/m³, bevorzugt 600 bis 1800 kg/m³, besonders bevorzugt 700 bis 1500 kg/m³, jeweils bestimmt in einem Stampfvolumeter STAV2003 der Firma JEL, wobei 2000 mal gestampft wurde, aufweist. Erfindungsgemäß bevorzugt weist das Zirkoniumoxid-Trägermaterial des im Festbett eingesetzten Katalysators eine Porengrößenverteilung auf, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

Die vorliegende Erfindung betrifft daher bevorzugt das erfindungsgemäße Verfahren, dadurch gekennzeichnet, dass das Zirkoniumoxid-Trägermaterial des im Festbett eingesetzten Katalysators eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

Erfindungsgemäß bevorzugt weist das Zirkoniumoxid-Trägermaterial des in Suspension eingesetzten Katalysators eine Porengrößenverteilung auf, bei der mehr als 40% der vorliegenden Poren Makroporen mit einem Durchmesser von > 50 nm sind, und der Rest zu 100% durch Mesoporen mit einem Durchmesser von 2 nm bis 50 nm gebildet werden.

Die vorliegende Erfindung betrifft daher bevorzugt das erfindungsgemäße Verfahren, dadurch gekennzeichnet, dass das Zirkoniumoxid-Trägermaterial des in Suspension eingesetzten Katalysators eine Porengrößenverteilung aufweist, bei der mehr als 40% der vorliegenden Poren Makroporen mit einem Durchmesser von > 50 nm sind, und der Rest zu 100% durch Mesoporen mit einem Durchmesser von 2 nm bis 50 nm gebildet werden.

Bevorzugt betrifft die vorliegende Erfindung das erfindungsgemäße Verfahren, wobei der Katalysator eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³, aufweist.

Bevorzugt betrifft die vorliegende Erfindung auch das erfindungsgemäße Verfahren, wobei der im Festbett eingesetzte Katalysator eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

Bevorzugt betrifft die vorliegende Erfindung auch das erfindungsgemäße Verfahren, wobei der in Suspension eingesetzte Katalysator eine Porengrößenverteilung aufweist, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

Der erfindungsgemäß eingesetzte Katalysator enthält das katalytisch aktive Metall Ruthenium besonders bevorzugt über das gesamte Trägermaterial, d.h. über den gesamten Durchmesser eines Trägermaterialteilchens verteilt, d. h. erfindungsgemäß ist das katalytisch aktive Ruthenium im Wesentlichen homogen über das gesamte Trägermaterial, d.h. über den gesamten Durchmesser eines Trägermaterialteilchens, verteilt vor.

In dem erfindungsgemäßen Verfahren wird im Allgemeinen eine Katalysatorbelastung von 0,01 bis 2 kg, bevorzugt 0,01 bis 1 kg, besonders bevorzugt 0,02 bis 0,6 kg organische, zu hydrierende Verbindung pro Liter Katalysator pro Stunde eingestellt. Eine gegebenenfalls während des erfindungsgemäßen Verfahrens stattfindende geringe Veränderung des erzielten Anteils an gewünschtem Produkt durch eine sich gegebenenfalls verändernde Aktivität des Katalysators im Laufe besonders langer Reaktionsperioden kann durch ein geringes Nachstellen der Reaktionstemperatur oder der anderen Parameter ausgeglichen werden. Die sich gegebenenfalls verändernden Anteile an gewünschtem Produkt können durch die Analytik des Reaktionsgemisches verfolgt werden. Diese Analytik kann durch dem Fachmann bekannte Methoden erfolgen, beispielsweise Gaschromatographie (GC).

Das erfindungsgemäße Verfahren kann im Allgemeinen so lange durchgeführt werden, bis ein geeigneter Umsatz erzielt worden ist. Wird das erfindungsgemäße Verfahren kontinuierlich durchgeführt, so entspricht die Reaktionsdauer der Verweilzeit der Reaktionsmischung im kontinuierlich betriebenen Reaktor. Erfindungsgemäß bevorzugt beträgt die Reaktionsdauer 10 bis 400 min.

Die vorliegende Erfindung betrifft daher bevorzugt das erfindungsgemäße Verfahren, wobei die Reaktionsdauer 10 bis 400 min beträgt.

Die erfindungsgemäß erhaltenen Hydriergemische können nach dem erfindungsgemäßen Verfahren gereinigt werden, beispielsweise durch Destillation. Gegebenenfalls im Reaktionsaustrag vorhandener Katalysator kann vor der Destillation entfernt werden, beispielsweise durch eine Fest-Flüssig-Trennung, wie Filtration, Sedimentation oder Zentrifugation. Lösungsmittel und nicht umgesetzte Ausgangsstoffe können in das Verfahren zurückgeführt werden.

Die erfindungsgemäß gewünschten Produkte werden nach erfolgreicher Aufarbeitung, beispielsweise durch Destillation, in einer Reinheit von mindestens 99 Gew.-% erhalten. In dieser Reinheit sind die genannten Verbindungen in der Regel für alle weiterverarbeitenden Prozesse einsetzbar.

Mit dem erfindungsgemäßen Verfahren ist es möglich, das gewünschte Produkt mit einem geringen Anteil des trans,trans-Isomeren zu erhalten. Dabei ist es erfindungsgemäß möglich, dass die gewünschte Isomerenverteilung durch die Hydrierung an sich erzielt wird, und dass die Isomerenverteilung bei einer gegebenenfalls durchgeführten destillativen Aufarbeitung zur Abtrennung von Lösungsmittel, nicht umgesetztem Edukt und gegebenenfalls gebildeten Nebenprodukten, nicht verändert werden muss.

Die vorliegende Erfindung betrifft auch die Verwendung eines Katalysators enthaltend Ruthenium auf einem Zirkoniumoxid-Trägermaterial zur Hydrierung von 4,4'-Methylendianilin (MDA), zu einer Mischung enthaltend die Isomeren von 4,4'-Diaminodicyclohexylmethan, wobei das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden Isomeren, vorliegt" wobei die Summe der vorliegenden Isomeren jeweils 100 Gew.-% ergibt, und/oder von Polymer-MDA, wobei als Produkt oligomere oder polymere, kernhydrierte Verbindungen erhalten werden, die als Widerholungseinheiten die Isomeren von 4,4'-Diaminodicyclohexylmethan enthalten, wobei das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden Isomeren, vorliegen, wobei die Summe der vorliegenden Isomeren jeweils 100 Gew.-% ergibt.

Des Weiteren betrifft die vorliegende Erfindung die Verwendung eines Katalysators enthaltend Ruthenium auf einem Zirkoniumoxid-Trägermaterial zur Hydrierung von 4,4'-Methylendianilin (MDA) zu einer Mischung enthaltend die Isomeren von 4,4'-Diaminodicyclohexylmethan und/oder von Polymer-MDA zu entsprechenden oligomeren oder polymeren, kernhydrierte Verbindungen mit einem Schmelzpunkt von weniger als 40 °C, bevorzugt weniger als 30 °C, besonders bevorzugt weniger als 22 °C. Eine bevorzugte Untergrenze für den Schmelzpunkt beträgt 0 °C.

Die durch das erfindungsgemäße Verfahren erhältlichen cycloaliphatischen Amine können als Synthesebaustein für die Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukte zur Herstellung quarternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulatoren und/oder als Ausgangssubstanzen für die Herstellung von Harnstoffen und Polyharnstoffen verwendet werden.

Insbesondere können die Hydrierprodukte von Bis-(4-aminophenyl)-methan (MDA) als Monomerbaustein für Polyamide verwendet werden.

Die vorliegende Erfindung betrifft daher des Weiteren auch die Verwendung einer Mischung enthaltend die Isomeren von 4,4'-Diaminodicyclohexylmethan, wobei das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden Isomeren, vorliegen, wobei die Summe der vorliegenden Isomeren immer 100 Gew.-% ergibt, als Synthesebausteine für die Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukte zur Herstellung quarternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen.

Bezüglich der einzelnen Merkmale und der bevorzugten Ausführungsformen dieser erfindungsgemäßen Verwendung gilt das bezüglich des erfindungsgemäßen Verfahrens Gesagte entsprechend.

Das erfindungsgemäße Verfahren und dessen Vorteile werden durch die folgenden Beispiele näher erläutert.

### Beispiele

### Herstellung der eingesetzten, erfindungsgemäßen Katalysatoren

### 1. Herstellung Festbettkatalysator 1 % Ru auf ZrO₂

238 g ZrO₂ Stränge (Ø 3 mm, SZ 31108 der Firma NorPro, BET-Oberfläche: 73 m²/g, Porenvolumen: 0,30 ml/g, Porenvolumenverteilung: 6% Makroporen, 94% Mesoporen) werden in einer Tränktrommel mit 19,81 g Ru(III)nitrosylnitratlösung (15,95 Gew.-% Ru(III)nitrosylnitrat (Fa Heraeus) in verdünnter Salpetersäure), verdünnt mit 35 ml VE-Wasser besprüht. Danach werden die Extrudate bei 120 °C für 16 h im Umlufttrockenschrank getrocknet und anschließend im Muffelofen für 2 h bei 180 °C kalziniert. Der Katalysator wird dann für 2 h bei 200 °C (4 l/h H₂; 40 l/h N₂) erst reduziert und mit einem Gemisch aus 10 Vol.-% Luft und 90 Vol.-% N₂ für 1 h bei Raumtemperatur passiviert. Die so hergestellte Aktivmasse enthält 1 Gew.-% Ru und 99 Gew.-% Zirkoniumoxid.

Der so hergestellte Katalysator weist folgende Charakteristika auf:
Eine BET-Oberfläche von 81 m²/g, eine Stampfdichte von 1,2 kg/L, ein Porenvolumen von 0,24 mL/g (bestimmt durch Hg-Porosimetrie).

### 2. Herstellung Suspensionskatalysator 10% Ru auf ZrO₂

30,51 g Ru(III)nitrosylnitratlösung (15,95 Gew% Ru(III)nitrosylnitrat (Fa. Heraeus) in verdünnter Salpetersäure) werden in einem Messzylinder vorgelegt und auf ein Gesamtvolumen von 37,5 ml mit VE-Wasser aufgefüllt. Danach werden 50 g Zirkoniumoxidpulver (D9-89, BASF, BET-Oberfläche: 78 m²/g, Porenvolumen: 0,84 ml/g, Porenvolumenverteilung: 68% Makroporen, 32% Mesoporen) in einer Keramikschale vorgelegt, die Lösung zugegeben und homogen gemischt. Anschließend wird das Pulver bei 120 °C im Umlufttrockenschrank für 16 h getrocknet und 2 h bei 200 °C unter Luft kalziniert. Dann wird das Pulver im Drehrohrofen erst 20 min mit 40 l/h N₂ gespült und danach innerhalb von 90 min reduziert (3 l/h Wasserstoff und 53 l/h Stickstoff). Nach Abkühlen auf Raumtemperatur wird der Wasserstoff abgestellt und mit ca. 60 l/h Stickstoff gespült. Zur Passivierung werden zunächst 60 l/h Stickstoff und 1 l/h Luft zugegeben und danach die Luftmenge langsam auf 10 l/h erhöht (0l/h Stickstoff). Dabei muss darauf geachtet werden, dass sich der Katalysator nicht über 35 °C erwärmt. Die so hergestellte Aktivmasse enthält 10 Gew.-% Ru und 90 Gew.-% ZrO₂. Der so hergestellte Katalysator weist folgende Charakteristika auf: Stampfdichte beträgt 1,13 kg/L, das Porenvolumen (Hg-Porosimetrie) beträgt 0,32 mL/g, die BET-Oberfläche beträgt 75 m²/g, die Porenverteilung ist wie folgt: 0% Mesoporen (2-50 nm), 100% Makroporen (>50 nm).

### Beispiel 1: Suspensionsfahrweise

Eine definierte Menge des erfindungsgemäßen Katalysators (10 Gew.-% Ru auf ZrO₂) wurde mit 7 ml einer 9 gew.-%igen Lösung von 4,4'-Methylendianilin (MDA) in Dioxan in einen Autoklaven mit einem Volumen von 10 ml gegeben. Die Reaktionsmischung wird anschließend unter 140 bar Wasserstoffdruck unter Rühren auf die entsprechende Reaktionstemperatur aufgeheizt und über einen definierten Zeitraum konstant gehalten. Danach wird die Lösung auf Raumtemperatur abgekühlt und der Autoklav auf Atmosphärendruck entspannt. Die Analyse der Reaktionsmischung erfolgt mittels GC Chromatographie, die Methode ist unten angegeben. Die Ergebnisse werden in den Tabellen 1 und 2 gezeigt:

**Tabelle 1**

| Nr. | T [°C] | Reaktionszeit [min] | Katalysatormenge [mg] | Umsatz [%] | Selektivität PACM [%] | Isomerenverhältnis [%] | | |
|---|---|---|---|---|---|---|---|---|
| | | | | | | trans/trans | cis/trans | cis/cis |
| 1.1 | 100 | 120 | 75 | 67 | 14 | 10 | 44 | 46 |
| 1.2 | 100 | 300 | 37,5 | 56 | 9 | 9 | 42 | 49 |
| 1.3 | 120 | 240 | 150 | 100 | 49 | 29 | 50 | 21 |
| 1.4 | 120 | 240 | 75 | 100 | 95 | 19 | 49 | 32 |
| 1.5 | 120 | 240 | 37,5 | 100 | 91 | 14 | 47 | 39 |
| 1.6 | 140 | 240 | 150 | 100 | 48 | 57 | 36 | 7 |
| 1.7 | 140 | 240 | 75 | 100 | 93 | 49 | 41 | 10 |
| 1.8 | 140 | 240 | 37,5 | 100 | 94 | 38 | 47 | 15 |

| | | | | | | | | |
|---|---|---|---|---|---|---|---|---|
| PACM bedeutet 4,4'-Diaminodicyclohexylmethan | | | | | | | | |

Die Ergebnisse der Tabelle 1 zeigen, dass bei einer Reaktionstemperatur von 120 °C das Produkt PACM mit Hilfe des erfindungsgemäßen Katalysators im erfindungsgemäßen Isomerenverhältnis erhalten wird. Ab 140 °C ändert sich das Isomerenverhältnis des Produktes dahingehend, dass der Anteil des trans,trans-Isomers signifikant ansteigt.

**Tabelle 2**

| Nr. | T [°C] | Reaktionszeit [min] | Katalysatormenge [mg] | Umsatz [%] | Selektivität PACM [%] | Isomerenverhältnis [%] | | |
|---|---|---|---|---|---|---|---|---|
| | | | | | | trans/trans | cis/trans | cis/cis |
| 1.9 | 120 | 120 | 75 | 99 | 85 | 14 | 46 | 40 |
| 1.10 | 120 | 180 | 75 | 91 | 85 | 16 | 49 | 35 |
| 1.11 | 120 | 240 | 75 | 100 | 95 | 19 | 49 | 32 |
| 1.12 | 140 | 120 | 75 | 100 | 93 | 35 | 48 | 17 |
| 1.13 | 140 | 180 | 75 | 100 | 94 | 45 | 44 | 11 |
| 1.14 | 140 | 240 | 75 | 100 | 93 | 49 | 41 | 10 |

| | | | | | | | | |
|---|---|---|---|---|---|---|---|---|
| PACM bedeutet 4,4'-Diaminodicyclohexylmethan | | | | | | | | |

Die Ergebnisse der Tabelle 2 zeigen, dass mit verlängerter Reaktionsdauer der Anteil an trans-trans Isomer im Produkt ansteigt.

### Beispiel 2: Fahrweise im Festbett

120 mL des passivierten erfindungsgemäßen Ruthenium-Trägerkatalysators (1 Gew.-% Ru auf ZrO₂) wurden in einen mit einem Außenmantel beheizten Rohrreaktor (1,4 m Höhe, 12 mm Innendurchmesser) gefüllt. Nachdem der Reaktor zunächst mit Wasserstoff geflutet wurde, wurde er im Anschluss mit einer Lösung von 10 Gew.-% 4,4'-Methylendianilin (MDA) beschickt. Es wurde bei variierender Temperatur und einem Druck von 140 bar hydriert, wobei die Katalysatorbelastung 0,04 kg MDA/kg Kat * h betrug, und der Reaktor mit Umlauf betrieben wurde, das heißt, dass ein Teil des Austrags in den Reaktor zurückgeführt wird. Die Reaktionsausträge wurden per Gaschromatographie analysiert und die Isomerenverteilung bestimmt. Die Methode wird unten angegeben. Die Ergebnisse werden in Tabelle 3 gezeigt und zeigen, dass durch den Einsatz des erfindungsgemäßen Katalysators bei 80 °C ein besonders niedriger Anteil des trans-trans-Isomers von 19% erreicht wird.

**Tabelle 3**

| Nr. | T [°C] | Umsatz [%] | Selektivität PACM [%] | Isomerenverhältnis | | |
|---|---|---|---|---|---|---|
| | | | | trans/trans | cis/trans | cis/cis |
| 2.1 | 140 | 96 | 95 | 51 | 40 | 9 |
| 2.2 | 130 | 94 | 96 | 47 | 41 | 12 |
| 2.3 | 100 | 84 | 86 | 28 | 49 | 23 |
| 2.4 | 80 | 67 | 60 | 19 | 49 | 32 |

| | | | | | | |
|---|---|---|---|---|---|---|
| PACM bedeutet 4,4'-Diaminodicyclohexylmethan | | | | | | |

### Beispiel 3: Fahrweise im Festbett

Es werden weitere erfindungsgemäße Umsetzungen durchgeführt. Die Ergebnisse werden in Tabelle 4 gezeigt

120 mL des passivierten erfindungsgemäßen Ruthenium-Trägerkatalysators (1 Gew.-% Ru auf ZrO₂) wurden in einem mit einem Außenmantel beheizten Rohrreaktor (1,4 m Höhe, 12 mm Innendurchmesser) gefüllt. Nachdem der Reaktor zunächst mit Wasserstoff geflutet wurde, wurde er im Anschluss mit einer Lösung von 10 Gew.-% 4,4'-Methylendianilin (MDA) beschickt. Es wurde bei variierender Temperatur und variierender Katalysatorbelastung, sowie einem Druck von 140 bar hydriert, wobei der Reaktor mit Umlauf betrieben wurde, das heißt, dass ein Teil des Austrags in den Reaktor zurückgeführt wird. Die Reaktionsausträge wurden per Gaschromatographie analysiert und die Isomerenverteilung bestimmt. Die Methode wird unten angegeben. Die Ergebnisse werden in Tabelle 3 gezeigt und zeigen, dass das Isomerenverhältnis durch die Wahl der Katalysatorbelastung und der Temperatur vorteilhaft beeinflusst werden kann.

**Tabelle 4**

| Nr. | Reaktionszeit [h] | Kat.-Belastung [kg/(kg*h)] | T [°C] | Umsatz [%] | Selektivität PACM [%] | Isomerenverhältnis | | |
|---|---|---|---|---|---|---|---|---|
| | | | | | | trans/trans | cis/trans | cis/cis |
| 3.1 | 1 - 149 | 0,04 | 140 | 95 | 95 | 51 | 40 | 9 |
| 3.2 | 364 - 477 | 0,04 | 80 | 67 | 60 | 19 | 49 | 33 |
| 3.3 | 1369 - 1377 | 0,02 | 77 | 78 | 73 | 20 | 49 | 31 |
| 3.4 | 1393 - 1424 | 0,02 | 60 | 47 | 32 | 18 | 48 | 34 |
| 3.5 | 1473 - 1539 | 0,02 | 90 | 85 | 86 | 22 | 49 | 29 |
| 3.6 | 1539 - 1561 | 0,04 | 90 | 72 | 74 | 21 | 49 | 30 |
| 3.7 | 1639 | 0,08 | 90 | 54 | 64 | 19 | 49 | 32 |

| | | | | | | | | |
|---|---|---|---|---|---|---|---|---|
| PACM bedeutet 4,4'-Diaminodicyclohexylmethan | | | | | | | | |

### Analytik per Gaschromatographie:

| | | |
|---|---|---|
| Säule: | 30m RTX5 Amin; 0,25mm; 0,5µm | |
| Temperaturprogramm: | 80°C - 0min - 20°C/min - 200°C - 0min - 4°C/min - 260°C - 5min | |
| | => 26 min Gesamtlaufzeit | |
| Retentionszeiten [min]: | trans,trans | 18,39 |
| | cis,trans | 18,58 |
| | cis,cis | 18,75 |
| | MDA (Edukt) | 25,00 |

## Patentansprüche

1. Verfahren zur Hydrierung von 4,4'-Methylendianilin und/oder Polymer-MDA mit Wasserstoff in Gegenwart eines Katalysators, **dadurch gekennzeichnet, dass** der Katalysator Ruthenium auf einem Zirkoniumoxid-Trägermaterial enthält.

2. Verfahren nach Anspruch 1, **dadurch gekennzeichnet, dass** es in Suspension oder im Festbett durchgeführt wird.

3. Verfahren nach Anspruch 1 oder 2, **dadurch gekennzeichnet, dass** es kontinuierlich oder diskontinuierlich durchgeführt wird.

4. Verfahren nach Anspruch 2 oder 3, **dadurch gekennzeichnet, dass** es im Festbett bei einer Temperatur von 50 bis 190 °C, bevorzugt 70 bis 120 °C, durchgeführt wird.

5. Verfahren nach Anspruch 2 oder 3, **dadurch gekennzeichnet, dass** es in Suspension bei einer Temperatur von 50 bis 190 °C, bevorzugt 100 bis 140 °C, durchgeführt wird.

6. Verfahren nach einem der Ansprüche 1 bis 5, **dadurch gekennzeichnet, dass** es bei einem Druck von 60 bis 300 bar durchgeführt wird.

7. Verfahren nach einem der Ansprüche 1 bis 6, **dadurch gekennzeichnet, dass** der Katalysator Ruthenium in einer Menge von 0,05 bis 15 Gew.-%, bezogen auf den gesamten Katalysator, enthält.

8. Verfahren nach einem der Ansprüche 1 bis 7, **dadurch gekennzeichnet, dass** das Zirkonoxid-Trägermaterial in monokliner, tetragonaler, kubischer, amorpher Phase oder einer Mischphase dieser Modifikationen vorliegt.

9. Verfahren nach einem der Ansprüche 1 bis 8, **dadurch gekennzeichnet, dass** das Zirkoniumoxid-Trägermaterial in monokliner, tetragonaler oder einer Mischphase dieser Modifikationen vorliegt.

10. Verfahren nach einem der Ansprüche 1 bis 9, **dadurch gekennzeichnet, dass** das Zirkonoxid-Trägermaterial eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³, aufweist.

11. Verfahren nach einem der Ansprüche 1 bis 10, **dadurch gekennzeichnet, dass** der Katalysator eine BET-Oberfläche von 30 bis 300 m²/g, bevorzugt 50 bis 90 m²/g, ein Porenvolumen von 0,1 bis 1 cm³/g, bevorzugt 0,1 bis 0,9 cm³/g, und eine Stampfdichte von 500 bis 2000 kg/m³, bevorzugt 700 bis 1750 kg/m³,aufweist.

12. Verfahren nach einem der Ansprüche 1 bis 11 **dadurch gekennzeichnet, dass** das Zirkoniumoxid-Trägermaterial des im Festbett eingesetzten Katalysators eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

13. Verfahren nach einem der Ansprüche 1 bis 12 **dadurch gekennzeichnet, dass** der im Festbett eingesetzte Katalysator eine Porengrößenverteilung aufweist, bei der mehr als 50% der vorliegenden Poren durch Mesoporen mit einem Durchmesser von 2 nm bis 50 nm, und der Rest zu 100% durch Makroporen mit einem Durchmesser > 50 nm gebildet werden.

14. Verfahren nach einem der Ansprüche 1 bis 11 **dadurch gekennzeichnet, dass** das Zirkoniumoxid-Trägermaterial des in Suspension eingesetzten Katalysators eine Porengrößenverteilung aufweist, bei der mehr als 40% der vorliegenden Poren Makroporen mit einem Durchmesser von > 50 nm sind, und der Rest zu 100% durch Mesoporen mit einem Durchmesser von 2 nm bis 50 nm gebildet werden.

15. Verfahren nach einem der Ansprüche 1 bis 11 **dadurch gekennzeichnet, dass** der in Suspension eingesetzte Katalysator eine Porengrößenverteilung aufweist, bei der mehr als 40% der vorliegenden Poren durch Makroporen mit einem Durchmesser von >50 nm, und der Rest zu 100% durch Mesoporen mit einem Durchmesser von 2 nm bis 50 nm gebildet werden.

16. Verfahren nach einem der Ansprüche 1 bis 15, **dadurch gekennzeichnet, dass** die Reaktionsdauer 10 bis 400 min beträgt.

17. Verfahren nach einem der Ansprüche 1 bis 16, **dadurch gekennzeichnet, dass** die Hydrierung in einem organischen Lösungsmittel erfolgt.

18. Verwendung eines Katalysators enthaltend Ruthenium auf einem Zirkoniumoxid-Trägermaterial zur Hydrierung von 4,4'-Methylendianilin und/oder Polymer-MDA.

19. Verwendung eines Katalysators enthaltend Ruthenium auf einem Zirkoniumoxid-Trägermaterial zur Hydrierung von 4,4'-Methylendianilin zu einer Mischung enthaltend die Isomeren von 4,4'-Diaminodicyclohexylmethan, wobei das trans,trans-Isomer in einer Menge von 10 bis 30 Gew.-%, das cis,trans-Isomer in einer Menge von 30 bis 55 Gew.-%, und das cis,cis-Isomer in einer Menge von 10 bis 50 Gew.-%, jeweils bezogen auf die Gesamtmenge an allen vorliegenden Isomeren, vorliegen, wobei die Summe der vorliegenden Isomeren immer 100 Gew.-% ergibt, und/oder von Polymer-MDA, wobei als Produkt oligomere oder polymere, kernhydrierte Verbindungen erhalten werden, die als Widerholungseinheiten die Isomeren von 4,4'-Diaminodicyclohexylmethan enthalten, wobei das trans,trans-Isomer in einer Menge von 10 bis 30 Gew.-%, das cis,trans-Isomer in einer Menge von 30 bis 55 Gew.-%, und das cis,cis-Isomer in einer Menge von 10 bis 50 Gew.-%, jeweils bezogen auf die Gesamtmenge an allen vorliegenden Isomeren, vorliegen wobei die Summe der vorliegenden Isomeren jeweils 100 Gew.-% ergibt.

20. Verwendung eines Katalysators enthaltend Ruthenium auf einem Zirkoniumoxid-Trägermaterial zur Hydrierung von 4,4'-Methylendianilin zu einer Mischung enthaltend die Isomeren von 4,4'-Diaminodicyclohexylmethan und/oder von Polymer-MDA zu entsprechenden oligomeren oder polymeren, kernhydrierte Verbindungen mit einem Schmelzpunkt von weniger als 40 °C, bevorzugt weniger als 30 °C, besonders bevorzugt weniger als 22 °C.

21. Verwendung einer Mischung enthaltend die Isomeren von 4,4'-Diaminodicyclohexylmethan, wobei das trans,trans-Isomer in einer Menge von 10 bis 30 Gew.-%, bevorzugt 10 bis 26 Gew.-%, das cis,trans-Isomer in einer Menge von 30 bis 55 Gew.-%, bevorzugt 40 bis 55 Gew.-%, und das cis,cis-Isomer in einer Menge von 10 bis 50 Gew.-%, bevorzugt 25 bis 40 Gew.-%, jeweils bezogen auf die Gesamtmenge an allen vorliegenden Isomeren, vorliegen, wobei die Summe der vorliegenden Isomeren immer 100 Gew.-% ergibt, als Synthesebausteine für die Herstellung von Tensiden, Arznei- und Pflanzenschutzmitteln, Stabilisatoren, Lichtschutzmitteln, Polymeren, Polyamiden, Isocyanaten, Härtern für Epoxyharze, Katalysatoren für Polyurethane, Zwischenprodukte zur Herstellung quarternärer Ammoniumverbindungen, Weichmachern, Korrosionsinhibitoren, Kunstharzen, Ionenaustauschern, Textilhilfsmitteln, Farbstoffen, Vulkanisationsbeschleunigern, Emulgatoren und/oder als Ausgangssubstanzen für die Herstellung von Harnstoffen und Polyharnstoffen.

## Claims

1. A process for hydrogenating 4,4'-methylenedianiline and/or polymeric MDA with hydrogen in the presence of a catalyst, wherein the catalyst comprises ruthenium on a zirconium oxide support material.

2. The process according to claim 1, which is carried out in suspension or in a fixed bed.

3. The process according to either of claims 1 and 2, which is carried out as a continuous operation or batchwise.

4. The process according to either of claims 2 and 3, which is carried out in a fixed bed at a temperature of from 50 to 190°C, preferably 70 to 120°C.

5. The process according to either of claims 2 and 3, which is carried out in suspension at a temperature of from 50 to 190°C, preferably 100 to 140°C.

6. The process according to any one of claims 1 to 5, which is carried out at a pressure of from 60 to 300 bar.

7. The process according to any one of claims 1 to 6, wherein the catalyst comprises ruthenium in an amount of from 0.05 to 15 wt% based on the whole catalyst.

8. The process according to any one of claims 1 to 7, wherein the zirconium oxide support material is present in monoclinic, tetragonal, cubic or amorphous phase or in a mixed phase of these modifications.

9. The process according to any one of claims 1 to 8, wherein the zirconium oxide support material is present in monoclinic or tetragonal phase or in a mixed phase of these modifications.

10. The process according to any one of claims 1 to 9, wherein the zirconium oxide support material has a BET surface area of from 30 to 300 m²/g, preferably 50 to 90 m²/g, a pore volume of from 0.1 to 1 cm³/g, preferably 0.1 to 0.9 cm³/g, and a tamped density of from 500 to 2000 kg/m³, preferably 700 to 1750 kg/m³.

11. The process according to any one of claims 1 to 10, wherein the catalyst has a BET surface area of from 30 to 300 m²/g, preferably 50 to 90 m²/g, a pore volume of from 0.1 to 1 cm³/g, preferably 0.1 to 0.9 cm³/g, and a tamped density of from 500 to 2000 kg/m³, preferably 700 to 1750 kg/m³.

12. The process according to any one of claims 1 to 11, wherein the zirconium oxide support material of the catalyst used in the fixed bed has a pore size distribution where more than 50% of the pores present are formed by mesopores having a diameter of from 2 nm to 50 nm and the remainder to 100% are formed by macropores having a diameter of > 50 nm.

13. The process according to any one of claims 1 to 12, wherein the catalyst used in the fixed bed has a pore size distribution where more than 50% of the pores present are formed by mesopores having a diameter of from 2 nm to 50 nm and the remainder to 100% are formed by macropores having a diameter of > 50 nm.

14. The process according to any one of claims 1 to 11, wherein the zirconium oxide support material of the catalyst used in suspension has a pore size distribution where more than 40% of the pores present are macropores having a diameter of > 50 nm and the remainder to 100% are formed by mesopores having a diameter of from 2 nm to 50 nm.

15. The process according to any one of claims 1 to 11, wherein the catalyst used in suspension has a pore size distribution where more than 40% of the pores present are formed by macropores having a diameter of > 50 nm and the remainder to 100% are formed by mesopores having a diameter of from 2 nm to 50 nm.

16. The process according to any one of claims 1 to 15, wherein the reaction time is from 10 to 400 min.

17. The process according to any one of claims 1 to 16, wherein the hydrogenation is carried out in an organic solvent.

18. The use of a catalyst comprising ruthenium on a zirconium oxide support material for the hydrogenation of 4,4'-methylenedianiline and/or polymeric MDA.

19. The use of a catalyst comprising ruthenium on a zirconium oxide support material for hydrogenating 4,4'-methylenedianiline to form a mixture comprising the isomers of 4,4'-diaminodicyclohexylmethane, wherein said mixture comprises the trans,trans isomer in an amount of from 10 to 30 wt%, the cis,trans isomer in an amount of from 30 to 55 wt% and the cis,cis isomer in an amount of from 10 to 50 wt%, in each case based on the total amount of all isomers present, wherein the sum of the isomers present always totals 100 wt%, and/or for hydrogenating polymeric MDA, wherein oligomeric or polymeric ring-hydrogenated compounds comprising the isomers of 4,4'-diaminodicyclohexylmethane as repeating units are obtained as product, wherein said product comprises the trans,trans isomer in an amount of from 10 to 30 wt%, the cis,trans isomer in an amount of from 30 to 55 wt% and the cis,cis isomer in an amount of from 10 to 50 wt%, in each case based on the total amount of all isomers present, wherein the sum of all isomers present in each case totals 100 wt%.

20. The use of a catalyst comprising ruthenium on a zirconium oxide support material for hydrogenating 4,4'-methylenedianiline to form a mixture comprising the isomers of 4,4'-diaminodicyclohexylmethane and/or for hydrogenating polymeric MDA to form corresponding oligomeric or polymeric ring-hydrogenated compounds having a melting point of less than 40°C, preferably less than 30°C, more preferably less than 22°C.

21. The use of a mixture comprising the isomers of 4,4'-diaminodicyclohexylmethane, wherein said mixture comprises the trans,trans isomer in an amount of from 10 to 30 wt%, preferably 10 to 26 wt%, the cis,trans isomer in an amount of from 30 to 55 wt%, preferably 40 to 55 wt%, and the cis,cis isomer in an amount of from 10 to 50 wt%, preferably 25 to 40 wt%, in each case based on the total amount of all isomers present, wherein the sum of all isomers present always totals 100 wt%, as synthetic building blocks for preparing surfactants, medicaments and crop protection agents, stabilizers including light stabilizers, polymers, polyamides, isocyanates, hardeners for epoxy resins, catalysts for polyurethanes, intermediates for preparing quaternary ammonium compounds, plasticizers, corrosion inhibitors, synthetic resins, ion exchangers, textile auxiliaries, dyes, vulcanization accelerants, emulsifiers and/or as starting substances for the preparation of ureas and polyureas.

## Revendications

1. Procédé d'hydrogénation de 4,4'-méthylène-dianiline et/ou de MDA polymère avec de l'hydrogène en présence d'un catalyseur, **caractérisé en ce que** le catalyseur contient du ruthénium sur un matériau support à base d'oxyde de zirconium.

2. Procédé selon la revendication 1, **caractérisé en ce qu'**il est réalisé en suspension ou dans un lit fixe.

3. Procédé selon la revendication 1 ou 2, **caractérisé en ce qu'**il est réalisé de manière continue ou discontinue.

4. Procédé selon la revendication 2 ou 3, **caractérisé en ce qu'**il est réalisé dans un lit fixe à une température de 50 à 190 °C, de préférence de 70 à 120 °C.

5. Procédé selon la revendication 2 ou 3, **caractérisé en ce qu'**il est réalisé en suspension à une température de 50 à 190 °C, de préférence de 100 à 140 °C.

6. Procédé selon l'une quelconque des revendications 1 à 5, **caractérisé en ce qu'**il est réalisé à une pression de 60 à 300 bar.

7. Procédé selon l'une quelconque des revendications 1 à 6, **caractérisé en ce que** le catalyseur contient du ruthénium en une quantité de 0,05 à 15 % en poids, par rapport à l'ensemble du catalyseur.

8. Procédé selon l'une quelconque des revendications 1 à 7, **caractérisé en ce que** le matériau support à base d'oxyde de zirconium se présente en une phase monoclinique, tétragonale, cubique, amorphe, ou une phase mixte de ces formes.

9. Procédé selon l'une quelconque des revendications 1 à 8, **caractérisé en ce que** le matériau support à base d'oxyde de zirconium se présente en une phase monoclinique, tétragonale, ou une phase mixte de ces formes.

10. Procédé selon l'une quelconque des revendications 1 à 9, **caractérisé en ce que** le matériau support à base d'oxyde de zirconium présente une surface BET de 30 à 300 m²/g, de préférence de 50 à 90 m²/g, un volume de pores de 0,1 à 1 cm³/g, de préférence de 0,1 à 0,9 cm³/g, et une densité tassée de 500 à 2 000 kg/m³, de préférence de 700 à 1 750 kg/m³.

11. Procédé selon l'une quelconque des revendications 1 à 10, **caractérisé en ce que** le catalyseur présente une surface BET de 30 à 300 m²/g, de préférence de 50 à 90 m²/g, un volume de pores de 0,1 à 1 cm³/g, de préférence de 0,1 à 0,9 cm³/g, et une densité tassée de 500 à 2 000 kg/m³, de préférence de 700 à 1 750 kg/m³.

12. Procédé selon l'une quelconque des revendications 1 à 11, **caractérisé en ce que** le matériau support à base d'oxyde de zirconium du catalyseur utilisé dans un lit fixe présente une distribution des tailles de pores telle que plus de 50 % des pores présents soient formés par des mésopores ayant un diamètre de 2 nm à 50 nm, et le reste jusqu'à 100 % par des macropores ayant un diamètre > 50 nm.

13. Procédé selon l'une quelconque des revendications 1 à 12, **caractérisé en ce que** le catalyseur utilisé dans un lit fixe présente une distribution des tailles de pores telle que plus de 50 % des pores présents soient formés par des mésopores ayant un diamètre de 2 nm à 50 nm, et le reste jusqu'à 100 % par des macropores ayant un diamètre > 50 nm.

14. Procédé selon l'une quelconque des revendications 1 à 11, **caractérisé en ce que** le matériau support à base d'oxyde de zirconium du catalyseur utilisé en suspension présente une distribution des tailles de pores telle que plus de 40 % des pores présents soient formés par des macropores ayant un diamètre > 50 nm, et le reste jusqu'à 100 % par des mésopores ayant un diamètre de 2 nm à 50 nm.

15. Procédé selon l'une quelconque des revendications 1 à 11, **caractérisé en ce que** le catalyseur utilisé en suspension présente une distribution des tailles de pores telle que plus de 40 % des pores présents soient formés par des macropores ayant un diamètre > 50 nm, et le reste jusqu'à 100 % par des mésopores ayant un diamètre de 2 nm à 50 nm.

16. Procédé selon l'une quelconque des revendications 1 à 15, **caractérisé en ce que** la durée de réaction est de 10 à 400 minutes.

17. Procédé selon l'une quelconque des revendications 1 à 16, **caractérisé en ce que** l'hydrogénation a lieu dans un solvant organique.

18. Utilisation d'un catalyseur contenant du ruthénium sur un matériau support à base d'oxyde de zirconium pour l'hydrogénation de 4,4'-méthylène-dianiline et/ou de MDA polymère.

19. Utilisation d'un catalyseur contenant du ruthénium sur un matériau support à base d'oxyde de zirconium pour l'hydrogénation de 4,4'-méthylène-dianiline en un mélange contenant les isomères de 4,4'-diaminodicyclohexylméthane, l'isomère trans,trans étant présent en une quantité de 10 à 30 % en poids, l'isomère cis,trans en une quantité de 30 à 55 % en poids et l'isomère cis,cis en une quantité de 10 à 50 % en poids, à chaque fois par rapport à la quantité totale de tous les isomères présents, la somme des isomères présents étant toujours de 100 % en poids, et/ou de MDA polymère, des composés oligomères ou polymères à noyau hydrogéné étant obtenus en tant que produit, qui contiennent en tant qu'unités de répétition les isomères de 4,4'-diaminodicyclohexylméthane, l'isomère trans,trans étant présent en une quantité de 10 à 30 % en poids, l'isomère cis,trans en une quantité de 30 à 55 % en poids, et l'isomère cis,cis en une quantité de 10 à 50 % en poids, à chaque fois par rapport à la quantité totale de tous les isomères présents, la somme des isomères présents étant à chaque fois de 100 % en poids.

20. Utilisation d'un catalyseur contenant du ruthénium sur un matériau support à base d'oxyde de zirconium pour l'hydrogénation de 4,4-méthylène-dianiline en un mélange contenant les isomères de 4,4'-diaminodicyclohexylméthane et/ou de MDA polymère en des composés oligomères ou polymères à noyau hydrogéné correspondants ayant un point de fusion inférieur à 40 °C, de préférence inférieur à 30 °C, de manière particulièrement préférée inférieur à 22 °C.

21. Utilisation d'un mélange contenant les isomères de 4,4'-diaminodicyclohexylméthane, l'isomère trans,trans étant présent en une quantité de 10 à 30 % en poids, de préférence de 10 à 26 % en poids, l'isomère cis,trans en une quantité de 30 à 55 % en poids, de préférence de 40 à 55 % en poids, et l'isomère cis,cis en une quantité de 10 à 50 % en poids, de préférence de 25 à 40 % en poids, à chaque fois par rapport à la quantité totale de tous les isomères présents, la somme des isomères présents étant toujours de 100 % en poids, en tant que constituant de synthèse pour la fabrication de tensioactifs, de médicaments et d'agents phytosanitaires, de stabilisateurs, de photoprotecteurs, de polymères, de polyamides, d'isocyanates, de durcisseurs pour résines époxy, de catalyseurs pour polyuréthanes, de produits intermédiaires pour la fabrication de composés d'ammonium quaternaires, de plastifiants, d'inhibiteurs de corrosion, de résines artificielles, d'échangeurs d'ions, d'adjuvants de textiles, de colorants, d'accélérateurs de vulcanisation, d'émulsifiants et/ou en tant que substances de départ pour la fabrication d'urées et de polyurées.
